# EUROPEAN PATENT SPECIFICATION

(11) **EP 4 401 740 B1**
(45) Date of publication and mention of the grant of the patent: **01.04.2026**
(21) Application number: 22868387.6
(22) Date of filing: 13.09.2022
(51) Int. Cl.: A61K 31/365, A61K 31/4162, A61K 31/575, A61K 31/56, A61K 31/59

(54) **USE OF TERPENOIDS IN THE TREATMENT OF MASTOCYTOMA**
VERWENDUNG VON TERPENOIDEN ZUR BEHANDLUNG VON MASTOZYTOM
UTILISATION DE TERPÉNOÏDES DANS LE TRAITEMENT D'UN MASTOCYTOME

(30) Priority: 13.09.2021 US 202163243392 P
(43) Date of publication of application: 24.07.2024
(73) Proprietor: Arjil Biotech Holding Company Limited, Hsinchu City 300044 (TW)
(72) Inventor: LO, Jir-Mehng, Hsinchu City 300044 (TW); LIANG, Hui-Ju, Hsinchu City 300044 (TW); LIN, Pei-Hsin, Hsinchu City 300044 (TW); WU, Yeh B, Reunion, Florida 34747-6412 (US)
(74) Representative: Straus, Alexander
(86) International application number: PCT/US2022/076346
(87) International publication number: WO 2023/039595

(56) References cited:
- MENG XIN ET AL: "Antitumor polysaccharides from mushrooms: a review on the structural characteristics, antitumor mechanisms and immunomodulating activities", CARBOHYDRATE RESEARCH, PERGAMON, GB, vol. 424, 2 March 2016 (2016-03-02), pages 30 - 41, XP029464445, ISSN: 0008-6215, DOI: 10.1016/J.CARRES.2016.02.008
- SHI L S ET AL: "Biologically active constituents from the fruiting body of Taiwanofungus camphoratus", BIOORGANIC & MEDICINAL CHEMISTRY, ELSEVIER, AMSTERDAM, NL, vol. 19, no. 1, 1 January 2011 (2011-01-01), pages 677 - 683, XP027577804, ISSN: 0968-0896, [retrieved on 20101020]
- HUANG YA-LING ET AL: "Antcin K, an Active Triterpenoid from the Fruiting Bodies of Basswood-Cultivated Antrodia cinnamomea , Inhibits Metastasis via Suppression of Integrin-Mediated Adhesion, Migration, and Invasion in Human Hepatoma Cells", JOURNAL OF AGRICULTURAL AND FOOD CHEMISTRY, vol. 63, no. 18, 5 May 2015 (2015-05-05), US, pages 4561 - 4569, XP093282896, ISSN: 0021-8561, DOI: 10.1021/jf5059304
- GANESAN NAGARAJAN, BASKARAN RATHINASAMY, VELMURUGAN BHARATH KUMAR, THANH NGUYEN CHI: "Antrodia cinnamomea —An updated minireview of its bioactive components and biological activity", JOURNAL OF FOOD BIOCHEMISTRY., WILEY-BLACKWELL PUBLISHING, INC., US, vol. 43, no. 8, 1 August 2019 (2019-08-01), US , pages 1 - 8, XP093046777, ISSN: 0145-8884, DOI: 10.1111/jfbc.12936
- BIBI S., ARSLANHAN M. D., LANGENFELD F., JEANNINGROS S., CERNY-REITERER S., HADZIJUSUFOVIC E., TCHERTANOV L., MORIGGL R., VALENT P: "Co-operating STAT5 and AKT signaling pathways in chronic myeloid leukemia and mastocytosis: possible new targets of therapy", HAEMATOLOGICA, FONDAZIONE FERRATA STORTI, IT, vol. 99, no. 3, 1 March 2014 (2014-03-01), IT , pages 417 - 429, XP093046778, ISSN: 0390-6078, DOI: 10.3324/haematol.2013.098442
- TU YUE-XING, WANG SHI-BING, FU LUO-QIN, LI SHUANG-SHUANG, GUO QIAN-PENG, WU YI, MOU XIAO-ZHOU, TONG XIANG-MIN: "Ovatodiolide targets chronic myeloid leukemia stem cells by epigenetically upregulating hsa-miR-155, suppressing the BCR-ABL fusion gene and dysregulating the PI3K/AKT/mTOR pathway", ONCOTARGET, IMPACT JOURNALS LLC, UNITED STATES, vol. 9, no. 3, 9 January 2018 (2018-01-09), United States , pages 3267 - 3277, XP093046780, ISSN: 1949-2553, DOI: 10.18632/oncotarget.23231

## Description

### FIELD OF THE INVENTION

The invention relates to herbal terpenoids from *Antrodia camphorata* and *Anisomeles indica* extract, particularly to a medicinal and edible formula to treat mast cell tumor effectively.

### BACKGROUND OF THE INVENTION

A mastocytoma or mast cell tumor is a type of round-cell tumor consisting of mast cells that occurs in many non-human animal species. If complete removal of the mast cell tumor through surgery is not possible due to the size or location, additional treatment, such as radiation therapy or chemotherapy, may be necessary. Vinblastine and lomustine are common chemotherapy agents used to treat dog mast cell tumors. Toceranib and masitinib, examples of receptor tyrosine kinase inhibitors, are used in the treatment of canine mast cell tumors. Both were recently approved by the U.S. Food and Drug Administration as dog-specific anticancer drugs. However, chemotherapeutic techniques have a range of adverse side effects that depend on the type of medications used. The use of medicinal plant-derived products to manage or arrest the carcinogenic process provides a safer alternative to the use of conventional chemodrug for treatment of the disease. Bibi S. et al. (Haematologica, (2014), 99(3), 417 - 429) discloses various treatments of mastocytosis such as antihistamines, glucocorticoids, cladribine, hydroxyurea etc.

Medicinal fungus *Antrodia camphorata* (AC) is a well-known Chinese folk medicine, known to possess numerous biological activities. To date, a total of 225 compounds have been isolated, identified, and structurally elucidated, including macromolecules (nucleic acids, proteins, and polysaccharides), small molecules (benzenoids, lignans, benzoquinones, and maleic/succinic acid derivatives), terpenoids (lanostane triterpenes, ergostane triterpenes, diterpenes, monoterpenes, and steroids), nucleotides (nucleobase and nucleoside), fatty acids, and fatty acid esters.

*Anisomeles indica* commonly known as 'Indian Catmint' is a source of medicinally active compounds and have various pharmacological effects. The plant is used traditionally as an analgesic, antiinflammatory and in skin problems. Further studies reveal the presence of various phytochemical constituents mainly triterpenes, β-sitosterol, stigmasterol, flavones, apigenin and ovatodiolides etc.

Plant terpenoids play a role in traditional herbal remedies and are also abundant in *Antrodia camphorate* and *Anisomeles indica.* However, the efficacy of these terpenoids and combination thereof in the treatment of mast cell tumor has not been evaluated.

### BRIEF DESCRIPTION OF THE DRAWINGS

**Figures 1** depict the effects of terpenoids on the cell viability on RBL-2H3 cells and the inhibition effect on β-hexosaminidase release.
**Figure 2** depicts the effects of terpenoids on the cell viability in RBL-2H3 cells.
**Figure 3** depicts the effects of terpenoids on the cell viability and the β-hexosaminidase release in RBL-2H3 cells.
**Figure 4** depicts the effects of terpenoids on the cell viability and the β-hexosaminidase release in RBL-2H3 cells.
**Figure 5** depicts the effects of combining terpenoids on P815 cell viability.
**Figures 6** depict the animals' body weight and tumor volume.
**Figures 7** depict the animals' tumor volume at 30th day.
**Figures 8** depict the animals' tumor weight at 30th day.
**Figures 9** depict the animals' tumor volume at 30th day.
**Figures 10** depict the photographs of tumor appearance at 23rd day.
**Figures 11** depicts H&E histological stain of tumor tissues.
**Figures 12** depicts H&E histological stain of tumor tissues (Mix-HD and AR101-DS2).

### DETAILED DESCRIPTION OF THE INVENTION

For the convenience of the description of the present invention, the central idea expressed in the above summary of the invention is expressed by way of specific examples. Various items in the embodiments are depicted in terms of ratios, dimensions, amounts of deformation, or displacements that are suitable for illustration, and are not drawn to the proportions of actual elements, as set forth above.

The terms "terpenoids" or "terpenes" refer to a large and diverse class of organic compounds, whose basic structure follows a general principle: 2-Methylbutane residues, less precisely but usually also referred to as isoprene units, (C₅)ₙ, build up the carbon skeleton of terpenes. About 30 000 terpenes are known at present in the literature. Depending on the number of 2-methylbutane (isoprene) subunits one differentiates between hemi- (C₅), mono- (C₁₀), sesqui- (C₁₅), di- (C₂₀), sester-(C₂₅), tri- (C₃₀), and tetraterpenes (C₄₀).

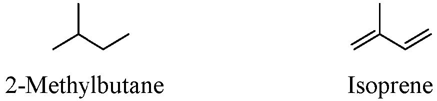

The terms "subject", "individual", "host", and "patient" are used interchangeably herein to refer to a living animal, including a human and a non-human animal. The Subject may, for example, be an organism possessing immune cells capable of responding to antigenic stimulation, and stimulatory and inhibitory signal transduction through cell Surface receptor binding. The Subject may be a mammal. Such as a human or non-human mammal, for example, dogs, cats, pigs, cows, sheep, goats, horses, rats, and mice. The term 'subject does not preclude individuals that are entirely normal with respect to a disease, or normal in all respects.

The term "treatment" refers to a therapeutic or preventative measure. The treatment may be administered to a subject having a medical disorder or who ultimately may acquire the disorder, in order to prevent, cure, delay, reduce the severity of or ameliorate one or more symptoms of a disorder or recurring disorder, or in order to prolong the Survival of a subject beyond that expected in the absence of such treatment.

The term "therapeutically effective amount' means the amount of the subject compound that may elicit a desired response, for example, a biological or medical response of a tissue, system or animal that is sought, for example, by a researcher, veterinarian, medical doctor, or other clinician.

### Cell culture

Mouse mastocytoma cells purchased from the Bioresource Collection and Research Center (Hsinchu City, Taiwan) and were cultured with Dulbecco's modified Eagle's (DMEM) medium (Gibco, Carlsbad, California, USA) that was supplemented with 10% fetal bovine serum (Gibco, Carlsbad, California, USA), 100 U/ml penicillin, and 100 µg/ml streptomycin.

### Cell counting kit-8 assay of cell viability

1×10⁵ cells per well of P815 cells were pretreated with terpenoids for 18h. 5 µL Cell Counting Kit-8 reagent was added to each well, incubating for 4 h followed by optical density determination at 450 nm by a microplate reader (Tecan Sunrise). The mean optical density (OD, absorbance) from four wells of the indicated group was used to calculate the percentage of cell viability as the formula as follows: [(ODₑₓₚₑᵣᵢₘₑₙₜ - OD_{blank})/(OD_{control} - OD_{blank})] × 100%, where ODₑₓₚₑᵣᵢₘₑₙₜ is the absorbance of cells treated with 0.1% DMSO or terpenoid-treated cells, and OD_{blank} is the absorbance of a well with medium only. OD_{control} is the absorbance of cells treated with 0.1% DMSO only.

### β-hexosaminidase Release Assay

RBL-2H3 cells were seeded into 24 well plate (0.5×10⁵ cells/0.5mL/well), and drug treatment was added every other day for 24 hours. On the third day, 500 µL of Anti-DNP-IgE (0.1 µg/mL) was added overnight. On the fourth day, add 160 µL DNP-BSA (0.1 µg/mL in Tyrode's buffer) to each well and react at 37°C for 1 hour. For each well in 24 wells, 50 µL of cell supernatant was mixed with 50 µL of P-NAG (triplicates), and 96 well plates were reacted at 37°C for 1 hour. Add 100 µL of stop buffer and measure the absorbance at 405 nm, which is the β-hexosaminidase data. For 24 well plates, add 300 µL of MTT reagent (0.5 mg/mL) to each well, react at 37°C for 2 hours, remove the supernatant, add 300 µL of DMSO to each well, and measure the absorbance at 570 nm. The data were calculated by one-way ANOVA with Dunnett's test (* p <0.5, **p <0.01, ***p <0.001, **** p <0.0001), and plotted with Graph pad.

### Animal Experiments

All animal experiments were approved by the Laboratory Animal Management and Ethics Committee of National Chung Hsing University, Taiwan. Cultured P815 cells (1×10⁵ cells in 0.2 mL of PBS) in 200 µL of extracellular matrix gel were subcutaneously injected into the back area of female BALB/c (6-week) athymic nude mice (weight, 20 ± 2 g). The mice were housed in a pathogen-free environment and then randomly divided into two groups for treatment (n = 5) as follows: the vehicle control (10% DMSO + 90% glyceryl trioctanoate) and Drug groups. The mice on both groups were daily administered via gavage feeding on the 6th day after transplantation. During the entire experimental period, the feed intake and motor activity of the mice were carefully observed, tumor sizes were measured with an electronic caliper every 3 days, and the tumor volume (mm³) was calculated according to the following formula: volume = (width² × length)/2. At the end of the study (day 20), all the mice were sacrificed by euthanization, and the tumors were quickly collected for weight measurement.

### Histopathological analysis

Mice were sacrificed on day 30, and tumors were removed, fixed with 10% formalin, 5% formic acid, and embedded in paraffin. Briefly, tumor sections were stained with hematoxylin and eosin (H&E) and analyzed by microscopy. Tissue sections were dewaxed with Xylene, and then treated with ethanol (100%, 95%, 75%, 50%). Tissues were stained with Hematoxylin for 1 minute and Eosin for 5 minutes.

### Statistical Analysis

All data are expressed as mean ± standard deviation. GraphPad Prism 5 (GraphPad Software Inc., San Diego, CA, USA) was used to analyze statistical differences among groups by one-way ANOVA, followed by Tukey's post-test, and unpaired two-tailed t-test was performed to analyze the differences between two independent groups. A p-value < 0.05 was considered as statistically significant.

### Example 1. Preparation of Antrodia camphorata extract

100 grams of *Antrodia camphorata* fruiting body is reflux with methanol for 6 hours, and the extract is collected and dried, thereby obtaining a total of 15 grams of methanol extract of *Antrodia camphorata.*

### Example 2. Preparation of active ingredients: Antcin K, dehydrosulphurenic acid/sulphurenic acid, Versisponic acid D and Dehydroeburicoic acid

The methanol extract of *Antrodia camphorata* is further separated by silica column chromatography using n-hexane / ethyl acetate / methanol as eluent to provide the fractions:

| Compound No. | Compound name and structure |
|---|---|
| AR101-DS1 | 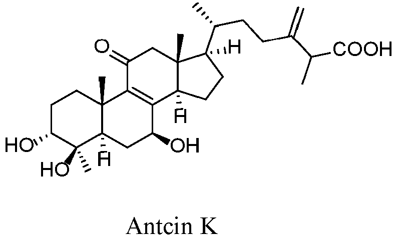 |
| AR101-DS2 | 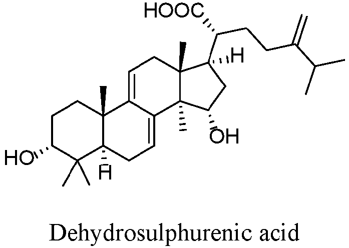 |
| | 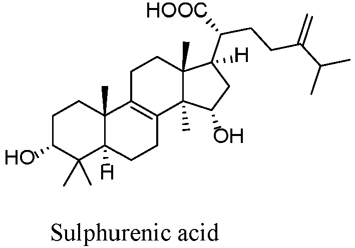 |
| AR101-DS3 | 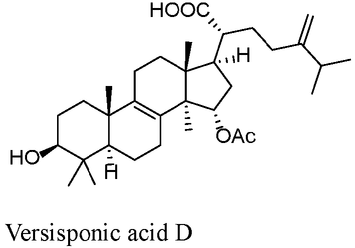 |
| AR101-DS4 | 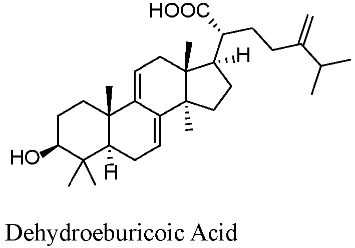 |

### Example 3. Preparation of active ingredients: AR100-DS1, AR100-DS4 ~ DS13

200 g of ethanol extract of *anisomeles indica* is taken, added into a silica-filled chromatographic column (10×15 cm), and subjected to a gradient elution with 1200 ml of each of the eluents: "n-hexane / ethyl acetate (with a ratio of 10/1, 5/1, 3/1, 1/1)", "hexane / ethyl acetate / methanol (with a ratio of 6/4/1, 3/2/1)" and "methanol" to obtain 140 g of an initial parting liquid.

The 140 g of the initial parting liquid is separated by using silica-filled chromatographic column (10×15 cm) and subjected to a gradient elution with 1000ml of each of the eluents: "dichloromethane", "dichloromethane/methanol (with a ratio of 10/1, 5/1, 7/3)" and "methanol" to obtain a series of separated concentrating substance.

| Compound No. | Compound name and structure |
|---|---|
| AR100-DS1 | 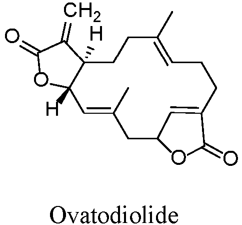 |
| AR100-DS4 | 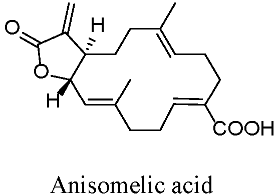 |
| AR100-DS5 | 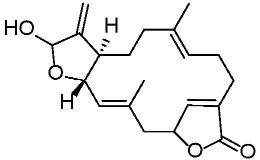 |
| AR100-DS7 | 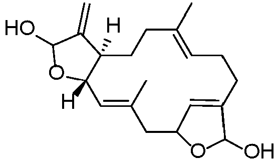 |
| AR100-DS8 | 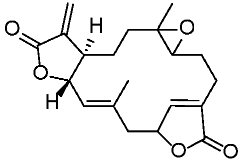 |
| AR100-DS9 | 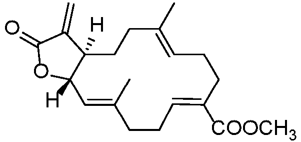 |
| AR100-DS10 | 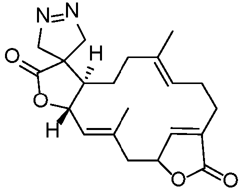 |
| AR100-DS11 | 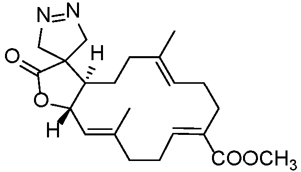 |
| AR100-DS12 | 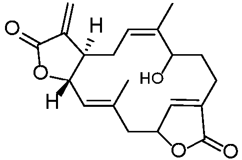 |

### Example 4. Effects of terpenoids on the cell viability on RBL-2H3 cells and the inhibition effect on β-hexosaminidase release

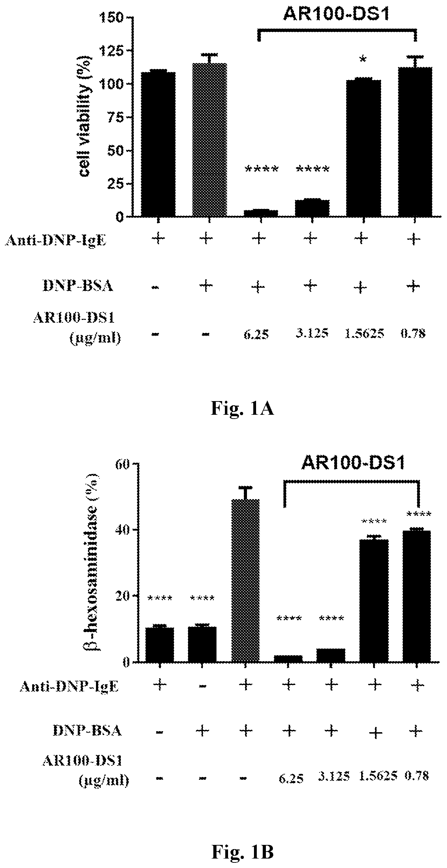

As shown in figures 1, under the safety dose, a significantly lower of β-hexosaminidase release was observed in AR100-DS1-treated groups. The results from triplicate determinations are presented as the percentage of viable cells. Statistical analysis was performed using one-way ANOVA (Dunnett's multiple comparisons test). Results are presented as the mean ± SD from at least three independent experiments. Statistical significance is indicated (ns: no significance, ** p < 0.01, *** p < 0.001, **** p < 0.0001, compared with DMSO).

### Example 5. Effects of terpenoids on the cell viability in RBL-2H3 cells

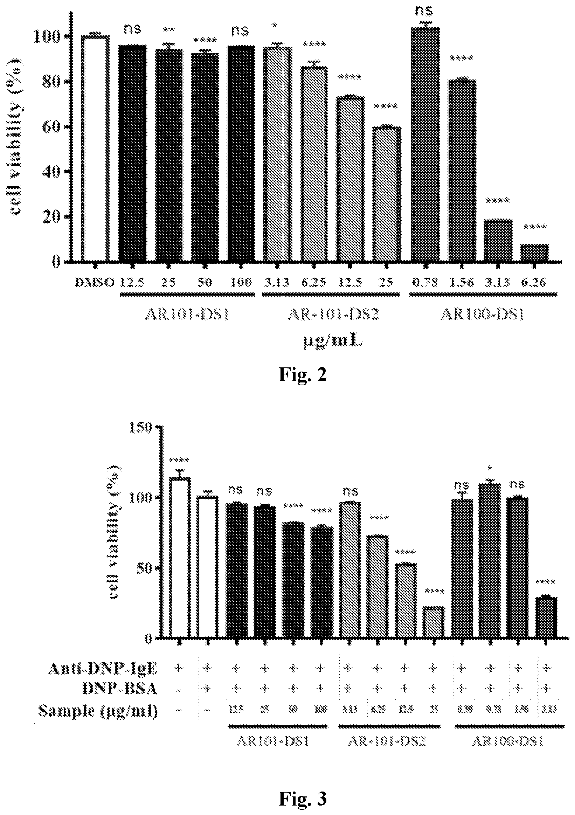

As shown in figure 2, the cell viability was determined using MTT assay. The results from triplicate determinations are presented as the percentage of viable cells. Statistical analysis was performed using one-way ANOVA (Dunnett's multiple comparisons test). Results are presented as the mean ± SD from at least three independent experiments. Statistical significance is indicated (ns: no significance, ** p < 0.01, *** p < 0.001, **** p < 0.0001, compared with DMSO).

### Example 6. Effects of terpenoids on the cell viability and the β-hexosaminidase release in RBL-2H3 cells

As shown in figure 3, the cell viability was determined using MTT assay. The results from triplicate determinations are presented as the percentage of viable cells. Statistical analysis was performed using one-way ANOVA (Dunnett's multiple comparisons test). Results are presented as the mean ± SD from at least three independent experiments. Statistical significance is indicated (ns: no significance, ** p < 0.01, *** p < 0.001, **** p < 0.0001, compared with DMSO).

### Example 7. Effects of terpenoids on the β-hexosaminidase release in RBL-2H3 cells

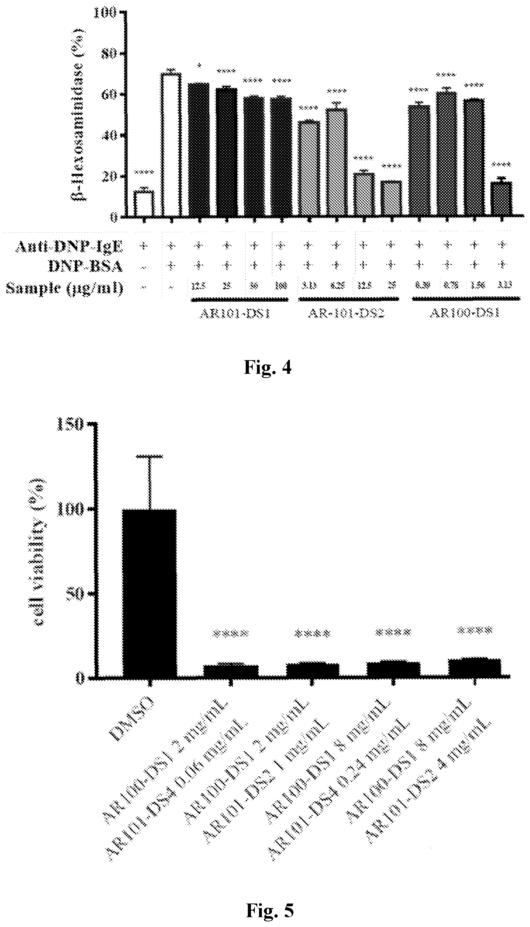

As shown in figure 4, the results from triplicate determinations are presented as the percentage of maximal antigen-induced degranulation. Statistical analysis was performed using one-way ANOVA (Dunnett's multiple comparisons test). Results are presented as the mean ± SD from at least three independent experiments. Statistical significance is indicated (ns: no significance, ** p < 0.01, *** p < 0.001, **** p < 0.0001, compared with DMSO).

### Example 8. Effects of terpenoids on the cell viability on P815 cells.

Mouse mastocytoma cells purchased from the Bioresource Collection and Research Center (Hsinchu City, Taiwan) and were cultured with Dulbecco's modified Eagle's (DMEM) medium (Gibco, Carlsbad, California, USA) that was supplemented with 10% fetal bovine serum (Gibco, Carlsbad, California, USA), 100 U/ml penicillin, and 100 µg/ml streptomycin. 1×10⁵ cells per well of P815 cells were pretreated with terpenoids for 18h. 5 µL Cell Counting Kit-8 reagent was added to each well, incubating for 4 h followed by optical density determination at 450 nm by a microplate reader (Tecan Sunrise). The mean optical density (OD, absorbance) from four wells of the indicated group was used to calculate the percentage of cell viability as the formula as follows: [(ODₑₓₚₑᵣᵢₘₑₙₜ - OD_{blank})/(OD_{control} - OD_{blank})] × 100%, where ODₑₓₚₑᵣᵢₘₑₙₜ is the absorbance of cells treated with 0.1% DMSO or terpenoids-treated cells, and ODblank is the absorbance of a well with medium only. OD_{control} is the absorbance of cells treated with 0.1% DMSO only.

As shown in figure 5, according to the study result, effects of these terpenoids or combination groups composed by terpenoids show significant inhibition ability of mast cancer cell P815, combination groups especially.

| Groups | Cell viability (%) Mean ± SD | p-value |
|---|---|---|
| DMSO | 100.00±9.38 | |
| AR100-DS1 1 mg/mL | 42.44±1.84 | **** |
| AR100-DS1 2 mg/mL | 39.52±3.09 | **** |
| AR100-DS1 3 mg/mL | 40.81±1.25 | **** |
| AR100-DS1 4 mg/mL | 43.33±0.65 | **** |
| AR100-DS1 5 mg/mL | 41.39±1.81 | **** |
| | | |
| AR101-DS2 0.25 mg/mL | 265.98±28.34 | **** |
| AR101-DS2 0.5 mg/mL | 130.20±12.98 | ns |
| AR101-DS2 0.75 mg/mL | 80.75±5.85 | ns |
| | | |
| AR101-DS4 0.02 mg/mL | 181.35±30.40 | **** |
| AR101-DS4 0.04 mg/mL | 69.27±4.58 | ns |
| AR101-DS4 0.06 mg/mL | 49.70±2.62 | ** |
| AR101-DS4 0.08 mg/mL | 54.29±1.85 | ** |
| AR101-DS4 0.1 mg/mL | 49.15±2.52 | ** |

| 5 Seeding 1×10 P815 | | Cell viability (%) Mean±SD | p-value |
|---|---|---|---|
| DMSO | | 100.00±31.11 | |
| AR100-DS1 2 mg/mL | AR101-DS4 0.06 mg/mL | 7.65±1.01 | **** |
| AR100-DS1 2 mg/mL | AR101-DS2 1 mg/mL | 8.13±0.85 | **** |
| | | | |
| AR100-DS1 8 mg/mL | AR101-DS4 0.24 mg/mL | 9.07±0.68 | **** |
| AR100-DS1 8 mg/mL | AR101-DS2 4 mg/mL | 10.59±0.57 | **** |

### Example 10. The effect of terpenoids administration on P815 cell development in nude mice

Male BALB/c mice are divided into the following groups:
1. Vehicle (10% DMSO+ 90% Olive oil)
2. AR100-DS1 (2 mg/kg)
3. AR100-DS1 (7.5 mg/kg)
4. AR100-DS1 (2 mg/kg) + AR101-DS4 (0.1 mg/kg)
5. AR100-DS1 (7.5 mg/kg) + AR101-DS4 (1 mg/kg)
6. AR101-DS4 (0.06 mg/kg)
7. AR101-DS4 (0.24 mg/kg)
8. AR101-DS2 (1 mg/kg)
9. AR101-DS4 (4 mg/kg)

The mice were administrated 100 µL samples intravenously every 2 days and sacrificed on the 30th day for histopathological examinations.

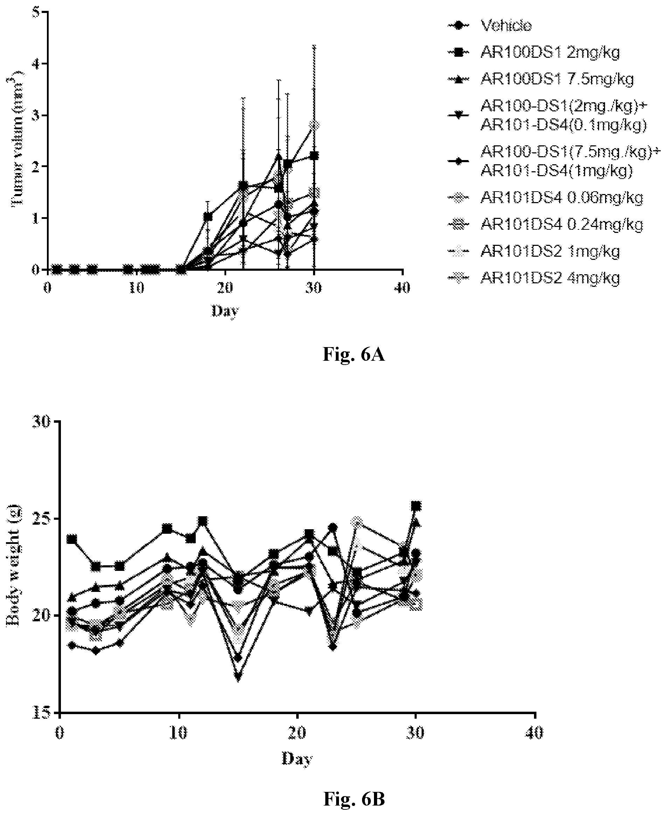

As shown in Figure 6A, comparing to the volume change of P815 mast cell tumor of the Vehicle group, these following groups: AR100-DS1+AR101-DS4 low dose, AR100-DS1+AR101-DS4 high dose, AR101-DS2 1 mg/kg and AR101-DS2 4 mg/kg, significantly reduced tumor growth was observed.

As shown in Figure 6B, the mean body weight over time curve. All groups of this study show no significant change of animals' body weight.

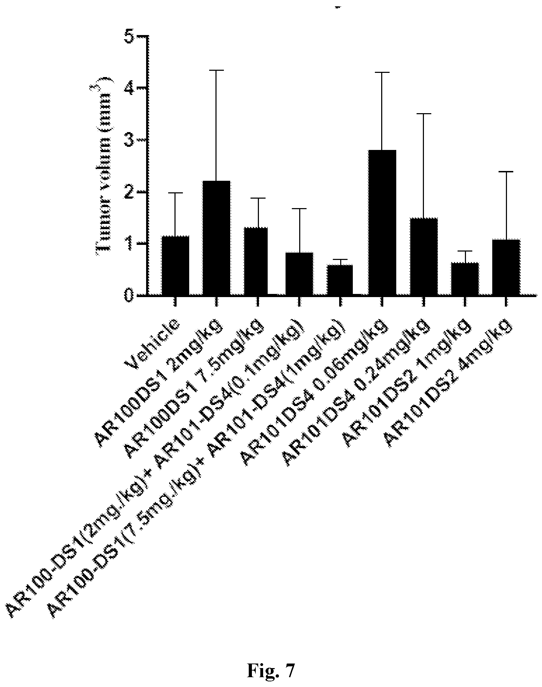

As shown in Figure 7, Comparing to the volume change of P815 mast cell tumor of the Vehicle group, these following groups: Mix low dose, Mix high dose, AR101-DS2 1 mg/kg and AR101-DS2 4 mg/kg , significantly reduced tumor growth was observed.

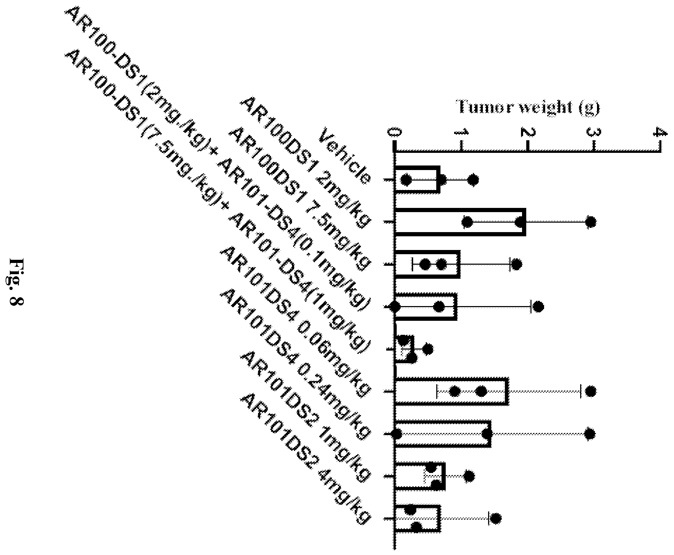

As shown in Figure 8, Per our study result, Mix HD inhibit the mast cell tumor growth significantly. According to the statistical analysis result standard deviation of Mix HD is quiet low, that means terpenoids show strong potential for treating the mast cell cancer.

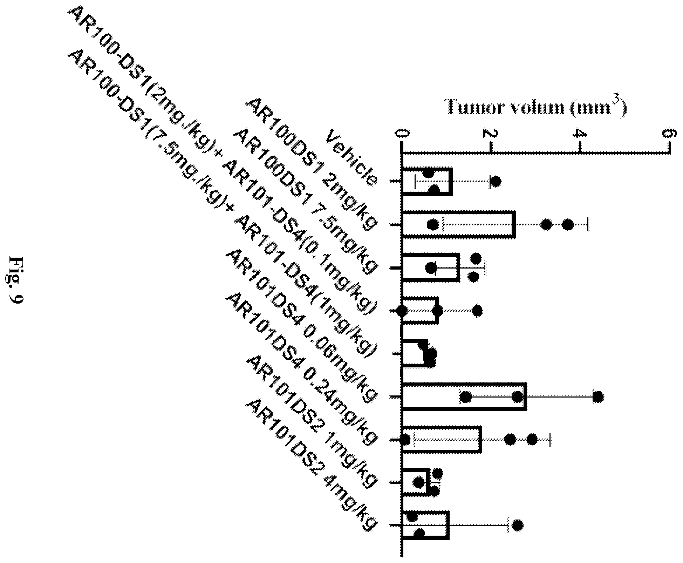

As shown in Figure 9, per our study result, Mix LD, Mix HD and AR101-DS2 1 mg/kg inhibit the mast cell tumor growth significantly. According to the statistical analysis result standard deviation of Mix HD and AR101-DS2 are quiet low, that means terpenoids show strong potential for treating the mast cell cancer.

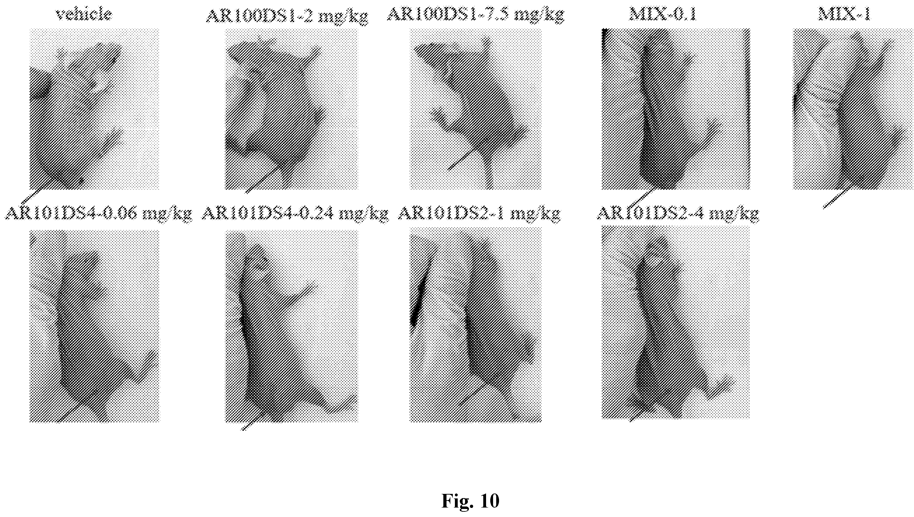

As shown in Figure 10, the P815 mast cell tumor was located in right rear side near the tail. Comparing to the tumor's volume change of Vehicle group, these following groups: Mix LD, Mix HD, AR101-DS2 1 mg/kg and AR101-DS2 4 mg/kg, significantly reduced tumor growth was observed.

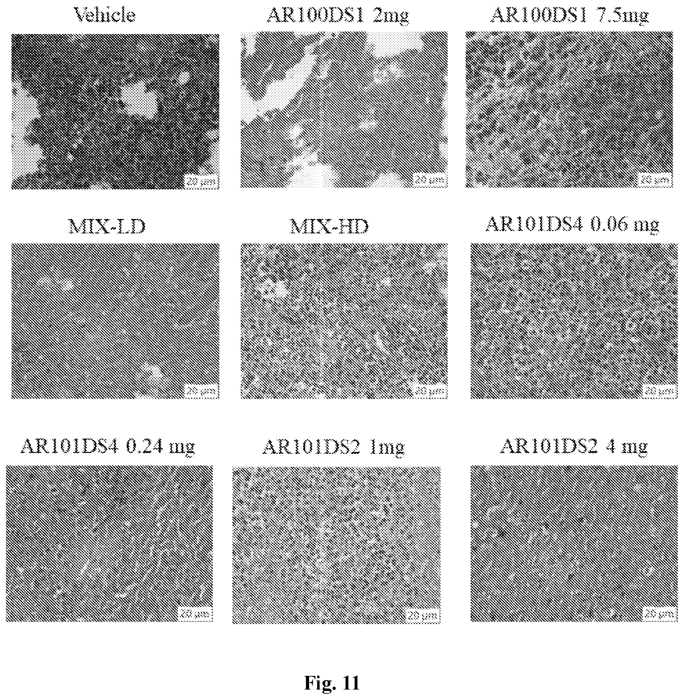

As shown in Figure 11, MIX-HD, AR101-DS4 0.06 mg and AR101-DS2 1 mg groups show their ability to arrest the tumor cell proliferation and cause the tumor cell nucleus aggregation. In this circumstance these processes maybe induced by cell apoptosis.

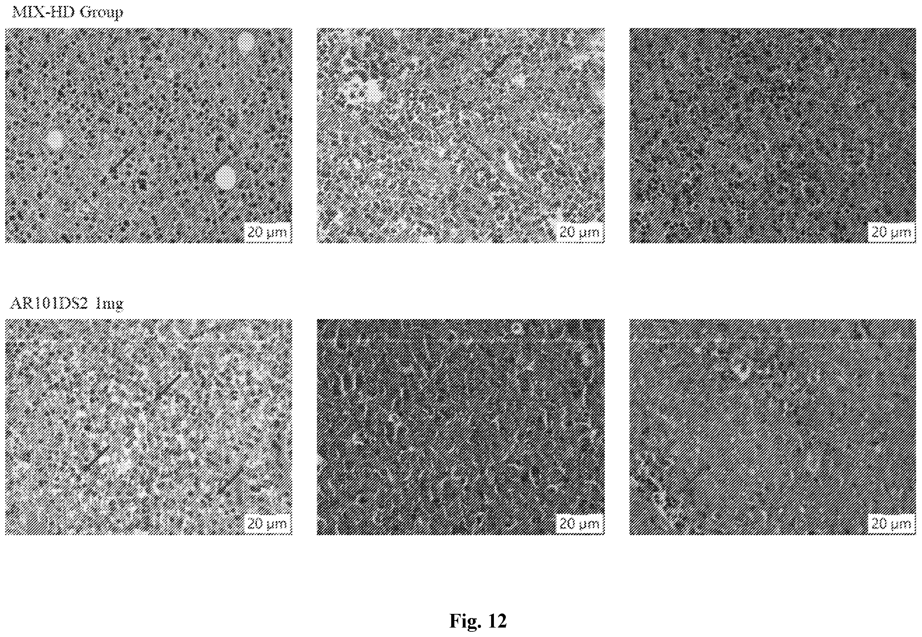

As shown in Figure 12, the Mix-HD and AR101DS2 1mg/kg groups arrest the tumor cell proliferation, cause the tumor cell nucleus aggregation, and cell to cell connection lost their tightness.

## Claims

1. A composition comprising at least one compound selected from the group consisting following formulae and combinations thereof: 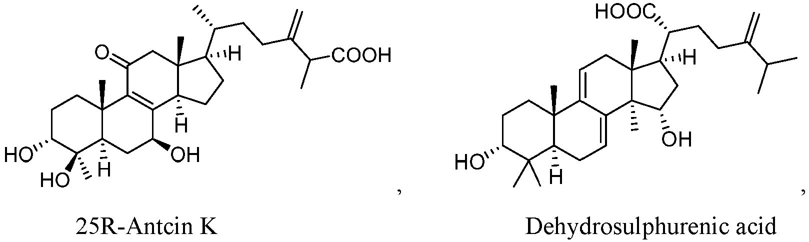 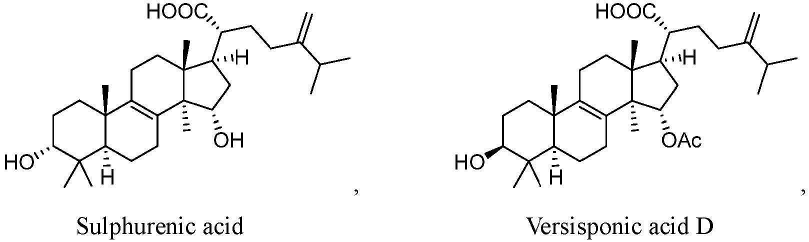 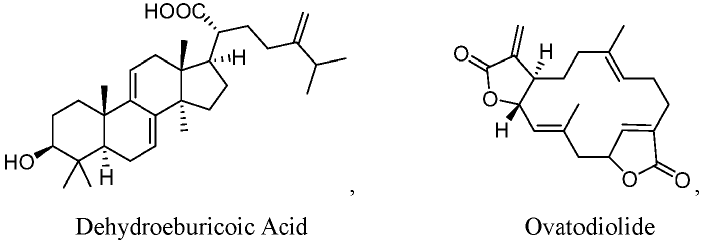 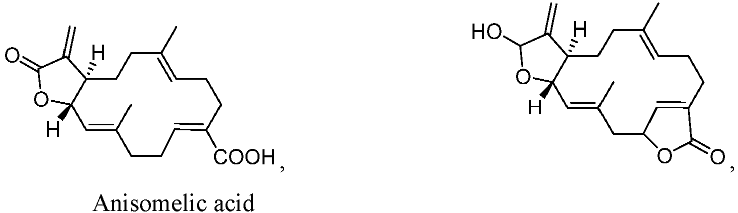 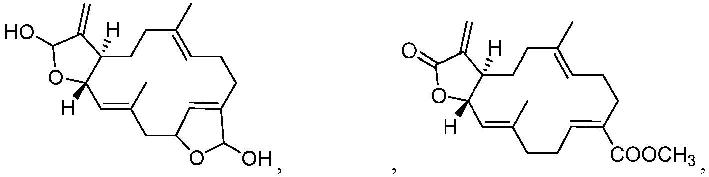 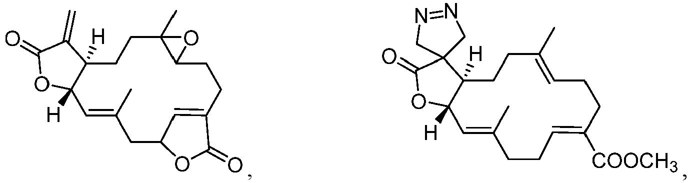 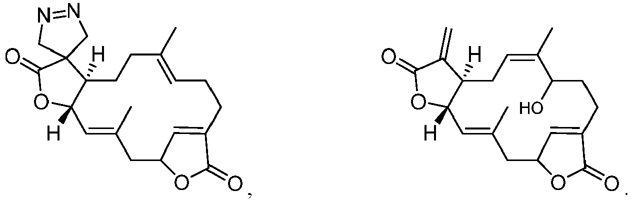 for use in a method of treating mastocytoma in a non-human animal.

2. The composition for use of claim 1, wherein the composition comprises:
( a) 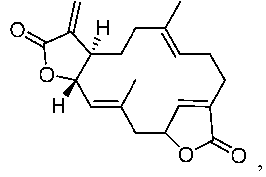 and
(b) 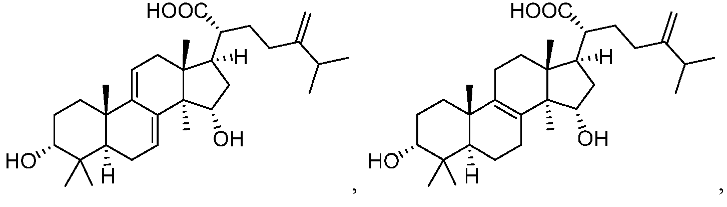 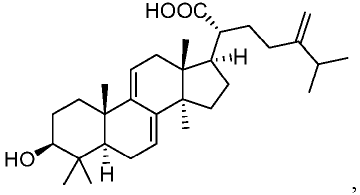 or combinations thereof.

3. The composition for use of claim 2, wherein the composition comprises:

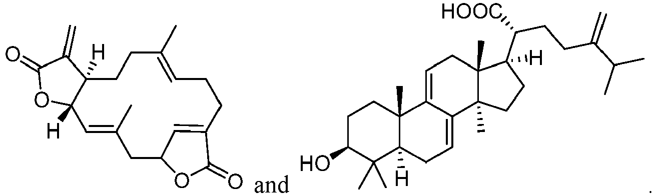

4. A composition, comprising
(a) 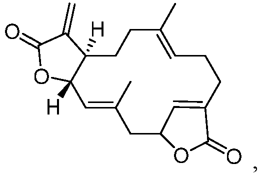 and
(b) 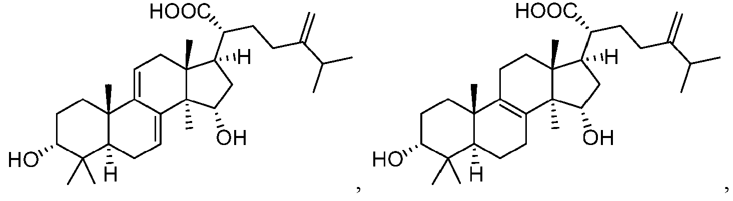 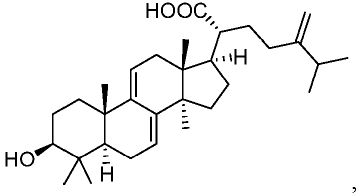 or combinations thereof.

5. The composition of claim 4, which comprises:

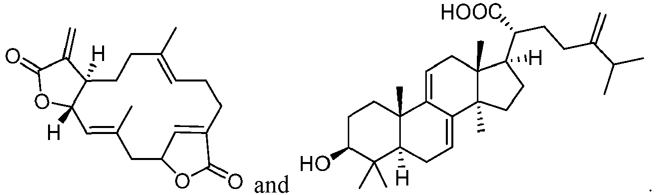

## Patentansprüche

1. Zusammensetzung, welche mindestens eine Verbindung umfasst, ausgewählt aus der Gruppe ist bestehend aus den Formeln und Kombinationen davon: 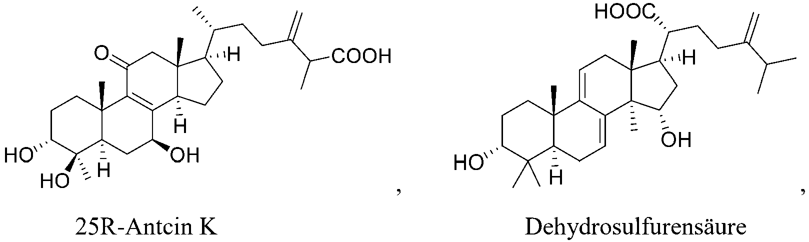 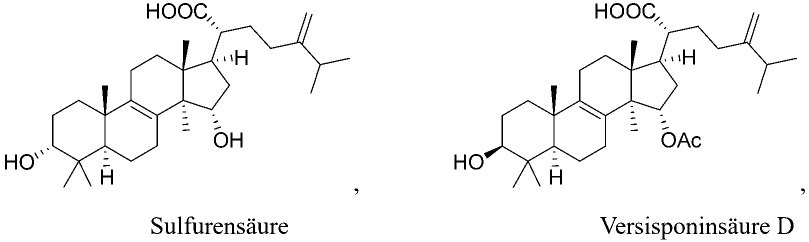 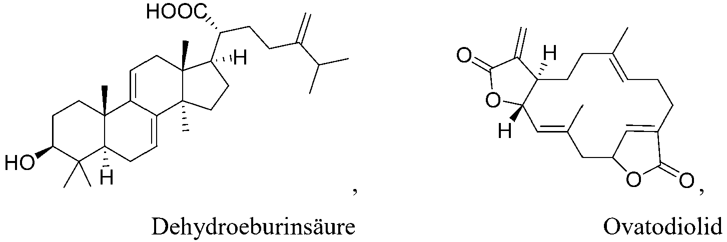 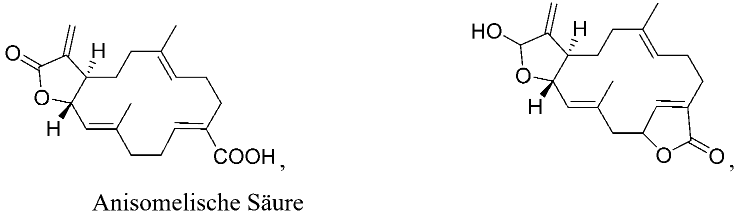 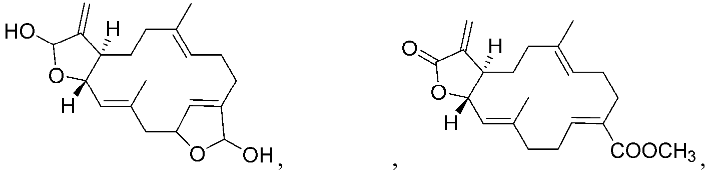 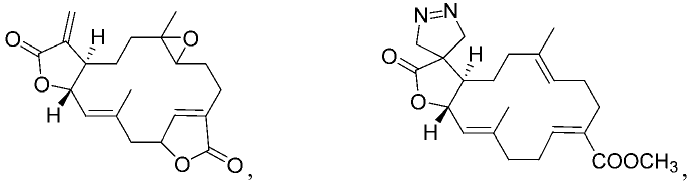 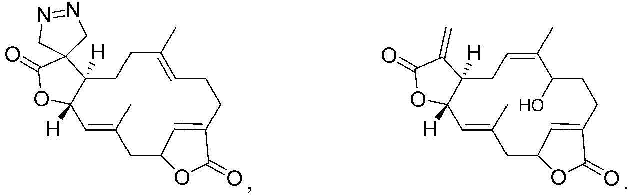 zur Verwendung in einem Verfahren zur Behandlung von Mastozytomen bei einem nicht-menschlichen Tier.

2. Zusammensetzung zur Verwendung nach Anspruch 1, worin die Zusammensetzung umfasst:
(a) 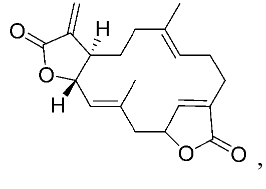 und
(b) 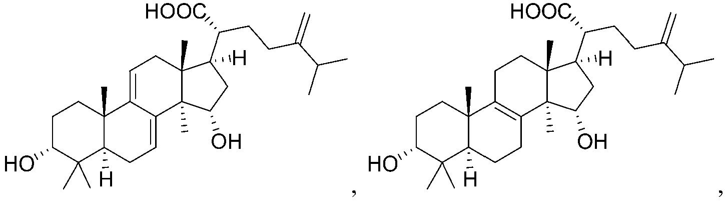 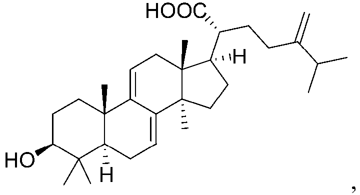 oder Kombinationen davon.

3. Zusammensetzung zur Verwendung gemäß Anspruch 2, worin die Zusammensetzung umfasst:

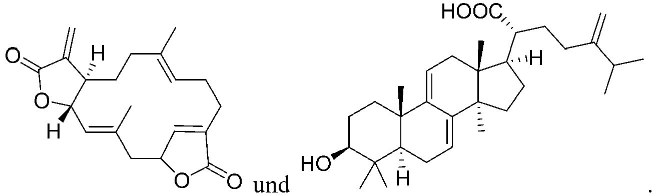

4. Zusammensetzung, welche umfasst:
(a) 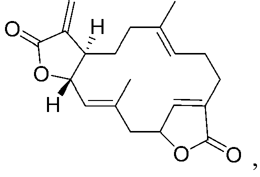 und
(b) 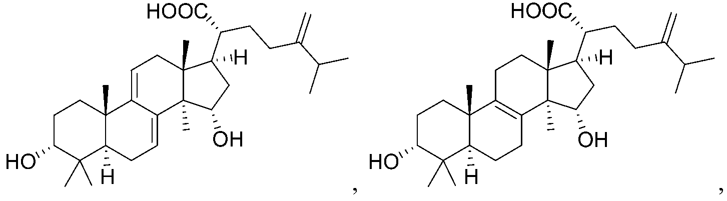 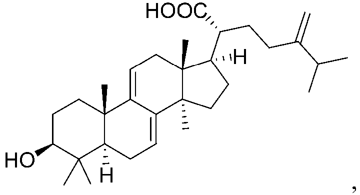 oder Kombinationen davon.

5. Zusammensetzung gemäß Anspruch 4, welche umfasst:

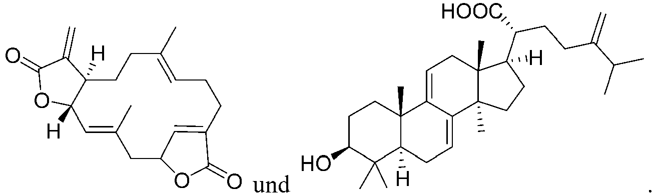

## Revendications

1. Composition comprenant au moins un composé choisi parmi le groupe constitué des formules suivantes et leurs combinaisons: 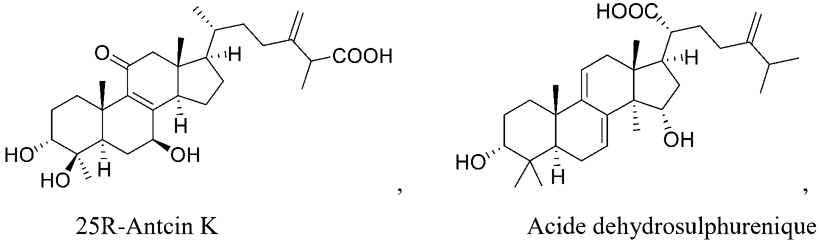 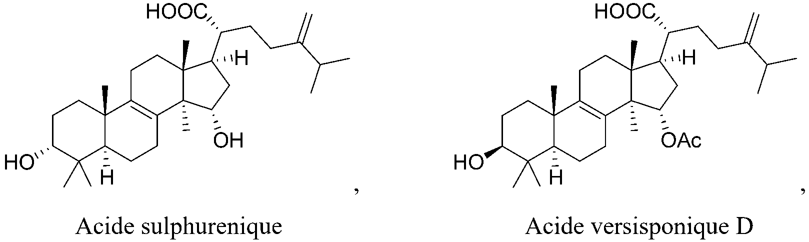 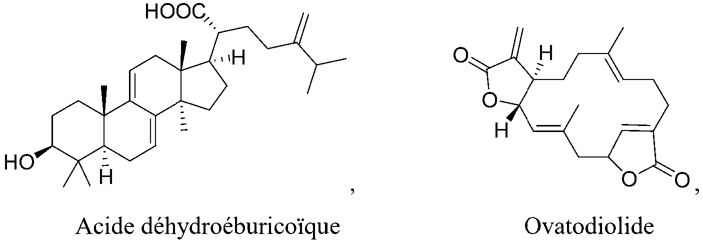 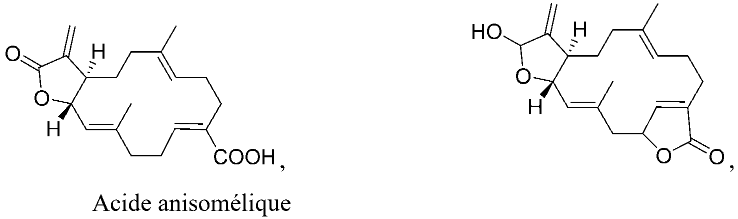 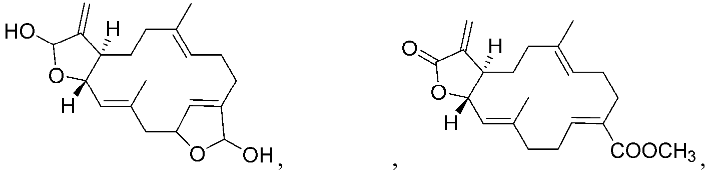 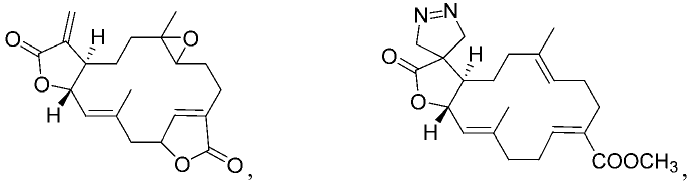 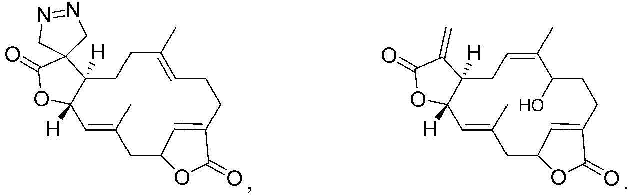 à être utilisé dans un procédé de traitement du mastocytome chez un animal non humain.

2. Composition selon la revendication 1, dans laquelle la composition comprend:
(a) 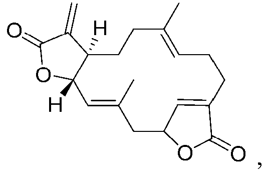 et
(b) 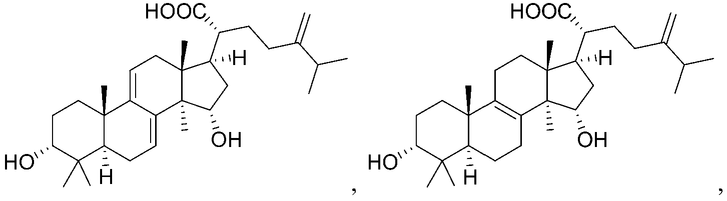 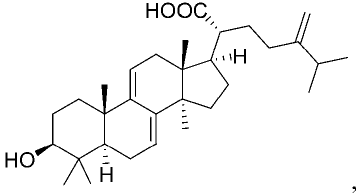 ou des combinaisons de ceux-ci.

3. Composition selon la revendication 2, dans laquelle la composition comprend:

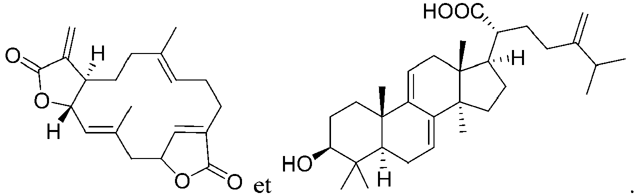

4. Composition comprenant:
(a) 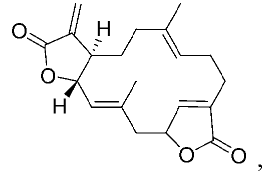 et
(b) 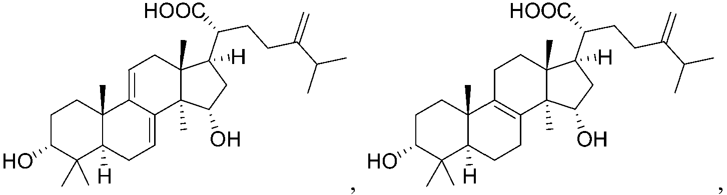 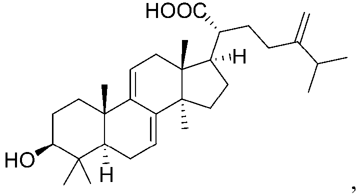 ou des combinaisons de ceux-ci.

5. Composition selon la revendication 4, comprenant:
